# EUROPEAN PATENT APPLICATION

(11) **EP 3 305 188 A1**
(43) Date of publication of application: **11.04.2018**
(21) Application number: 16774838.3
(22) Date of filing: 03.06.2016
(51) Int. Cl.: A61B 5/04, A61B 5/00, G06F 3/041, A61B 5/0402, A61B 5/021, A61B 5/024

(54) **BIOSIGNAL MEASUREMENT DEVICE UTILIZING PLURALITY OF ELECTRODES FOR MEASURING BIOSIGNAL AS TOUCH SENSORS**

(30) Priority: 04.06.2015 KR 20150079028
(71) Applicant: Huinno, Co., Ltd., Seoul 06249 (KR)
(72) Inventor: SHIN, Min Yong, Suwon-si Gyeonggi-do 16571 (KR)
(74) Representative: Glawe, Delfs, Moll
(86) International application number: PCT/KR2016/005903
(87) International publication number: WO 2016/195413

(57) **Abstract**

A biosignal measurement apparatus capable of measuring a user's biosignal in real time is provided, including a plurality of electrodes which are separated from each other and configured to measure a biosignal through contact with a user's body; and a sensing unit which is electrically connected to the electrodes and measures an electrical signal formed in a circuit connected to the electrodes, wherein the electrodes are also configured to be utilized as touch sensors for executing functions other than a biosignal measurement function of the biosignal measurement apparatus, and the biosignal measurement apparatus is configured to execute a different function according to whether the user's body has touched the electrodes and a period of time during which the user's body touches the electrodes.

## Description

### [Technical Field]

The present invention relates to a biosignal measurement apparatus configured to utilize a plurality of electrodes for biosignal measurement as touch sensors for executing other functions of the biosignal measurement apparatus, and more particularly, to a biosignal measurement apparatus configured to execute various functions thereof by sensing the contact of a user's body with a plurality of electrodes formed therein.

### [Background Art]

Recently, the rapid development of scientific technology has improved the quality of life of the entire humanity and caused a lot of changes in the medical environment. In the past, after a medical image, such as X-ray, computed tomography (CT), functional magnetic resonance imaging (fMRI), etc., was taken at a hospital, it took several hours to several days before the medical image could be interpreted.

However, from about ten years ago, a picture archiving and communication system (PACS) which sends a medical image taken directly to the monitor screen of a radiologist so that the radiologist can immediately interpret the medical image has been introduced. In addition, a lot of ubiquitous healthcare-related medical devices that enable a user to check his or her blood glucose and blood pressure without visiting a hospital have been developed and come into wide use.

In particular, high blood pressure is a major risk factor for various diseases, and its prevalence rate is increasing.

Therefore, there has been a growing need for a monitoring system that can continuously measure blood pressure and provide the measured blood pressure in real time. Accordingly, various types of research are being attempted to develop the monitoring system.

As one of these research efforts, the present inventor has invented a biosignal measurement apparatus (a wristwatch apparatus) capable of measuring various biosignals in real time and estimating a user's blood pressure in real time based on the measured biosignals. The wristwatch apparatus includes a first electrode for biosignal measurement on the back (an inner surface that contacts the wrist wearing the wristwatch apparatus) thereof and a second electrode for biosignal measurement on the front (an outer surface that does not contact the wrist wearing the wristwatch apparatus) thereof. In a state where the first electrode is in contact with the user's wrist wearing the wristwatch apparatus, if the user touches the second electrode with another part (such as a finger) of his or her body, the user's electrocardiogram (ECG) signal is measured by the first electrode and the second electrode. In addition, the wristwatch apparatus may include a measurement module capable of measuring photoplethysmography (PPG) and saturation of peripheral oxygen (SpO₂) signals or may be connected to the measurement module. The measurement module may observe a blood flow rate in peripheral blood vessels of a fingertip or a toe tip by irradiating light to the fingertip or the toe tip using a red light source and/or an infrared light source and measuring light transmitted or reflected by the human body using a photo sensor and may measure the PPG and SpO₂ signals based on the observed blood flow rate. In addition, the user's blood pressure can be estimated in real time using the ECG, PPG and SpO₂ signals.

The user's biosignals (ECG, PPG and SpO₂) mentioned above will now be described in greater detail below.

ECG is a waveform that represents the vector sum of action potentials generated by a special excitatory & conductive system of the heart. That is, the ECG is a vector sum signal, measured using electrodes attached onto the human body, of action potentials generated by components of the heart such as sinoatrial (SA) node, atrioventricular (AV) node, His bundle, bundle branches, Purkinje fibers, etc. For example, an ECG signal can be obtained using a standard limb lead method.

PPG is a pulse wave signal measured in peripheral blood vessels when blood ejected during ventricular systole is delivered to the peripheral blood vessels. A PPG signal can be measured using optical characteristics of biological tissue. For example, a photo sensor module that can measure a pulse wave signal may be attached to a location (such as a fingertip or a toe tip) where the peripheral blood vessels are distributed. Then, the photo sensor module may measure PPG singal by converting a change (a volume change) in the blood flow rate of the peripheral blood vessels into a change in the amount of light. The PPG signal can be measured by irradiating red light generated by a light-emitting unit of the photo sensor module to the human body and observing a change in the amount of light reflected by the human body and then received by a light receiving unit. Information such as pulse transit time (PTT) or pulse wave velocity (PWV) is extracted by analyzing the correlation between a PPG signal and an ECG signal, instead of using only the PPG signal, and cardiovascular diseases are diagnosed based on the extracted information. For example, after a characteristic point is obtained by performing a quadratic differential on a PPG signal, PTT and PWV signals may be extracted by measuring a time interval from a peak (R wave) of an ECG signal. Then, the extracted PTT and PWV signals may be used to diagnose the state of blood vessels, hardening of the arteries, peripheral circulatory disturbance, etc.

SpO₂ is a biosignal indicating oxygen content in hemoglobin from among various components of blood. SpO₂ can be measured by sequentially irradiating red light and infrared light to an area of peripheral blood vessels of the human body in each period and observing a change in the amount of light reflected by the human body and then received by a light receiving unit. For example, Sp0₂ can be measured using the PPG sensor module (the photo sensor module) described above.

However, while the conventional biosignal measurement apparatus provides a useful function of measuring a user's biosignal in real time, it utilizes components (a plurality of electrodes) formed therein for biosignal measurement only for the purpose of biosignal measurement. Therefore, the utilization of the components is not high.

### [Disclosure]

### [Technical Problem]

The present invention has been made to solve the foregoing problems of the prior art and therefore an aspect of the present invention is to improve utilization of components (a plurality of electrodes) included in a biosignal measurement apparatus for biosignal measurement by adding additional functions to the electrodes.

### [Technical Solution]

According to an aspect of the present invention, there is provided a biosignal measurement apparatus capable of measuring a user's biosignal in real time, the biosignal measurement apparatus comprising: a plurality of electrodes which are separated from each other and configured to measure a biosignal through contact with a user's body; and a sensing unit which is electrically connected to the electrodes and measures an electrical signal formed in a circuit connected to the electrodes, wherein the electrodes are also configured to be utilized as touch sensors for executing functions other than a biosignal measurement function of the biosignal measurement apparatus, and the biosignal measurement apparatus is configured to execute a different function according to whether the user's body has touched the electrodes and a period of time during which the user's body touches the electrodes.

According to an aspect of the present invention, the biosignal measurement apparatus, being configured to utilize the electrodes as touch sensors by determining whether the user's body has touched the electrodes, based on whether the electrical signal has been sensed by the sensing unit.

According to an aspect of the present invention, the electrodes of the biosignal measurement apparatus are configured to function as electrodes for biosignal measurement in a state where a biosignal measurement program is running in the biosignal measurement apparatus and to function as touch sensors for executing functions other than biosignal measurement in a state where the biosignal measurement program is not running.

According to an aspect of the present invention, the biosignal measurement apparatus, being configured to execute a first function other than the biosignal measurement program when the user's body touches the electrodes for less than a first reference time in the state where the biosignal measurement program is not running, a second function other than the biosignal measurement program when the user's body touches the electrodes for the first reference time or longer and less than a second reference time in the state where the biosignal measurement program is not running, and the biosignal measurement program when the user's body touches the electrodes for the second reference time or longer in the state where the biosignal measurement program is not running.

The first reference time, the second reference time, the first function, and the second function are configured to be set and modified by the user.

According to an aspect of the present invention, the biosignal measurement apparatus, being configured to determine a degree to which the user's body touches the electrodes and, when determining that the degree to which the user's body touches the electrodes is outside a range for biosignal measurement, inform the user of the determination result.

According to an aspect of the present invention, the biosignal measurement apparatus, being a wristwatch apparatus which can be worn around the user's wrist, wherein the electrodes comprise a first electrode formed on a surface which contacts the user's wrist wearing the biosignal measurement apparatus and a second electrode disposed on a different surface from the first electrode.

The second electrode is disposed on a display screen of a display module formed on a front of the biosignal measurement apparatus.

According to an aspect of the present invention, the biosignal measurement apparatus, being configured to correct a biosignal based on the assumption that the biosignal was measured in a state where the first electrode A was in contact with the user's wrist and where the second electrode B was in contact with the user's finger.

The electrodes form an electrocardiogram (ECG) sensor module for measuring the user's ECG signal.

The sensing unit is configured to sense the electrical signal by converting a value of a voltage or an electric current generated by the human body as it is or by setting a threshold and converting the voltage or the electric current generated by the human body according to a range.

### [Advantageous Effects]

According to the present invention, additional functions other than biosignal measurement are added to a plurality of electrodes included in a biosignal measurement apparatus for biosignal measurement. Therefore, utilization of the components is increased.

In addition, according to the present invention, the electrodes formed in the biosignal measurement apparatus are utilized as touch sensors. Therefore, various functions of the biosignal measurement apparatus can be executed without touch sensors.

Furthermore, according to the present invention, various functions of the biosignal measurement apparatus are performed according to a length of time during which a user's body touches the electrodes formed in the biosignal measurement apparatus. Therefore, more various functions can be executed without additional touch sensors.

Also, according to the present invention, it is determined whether the user's body has touched the electrodes formed in the biosignal measurement apparatus to an appropriate degree for biosignal measurement, and the determination result is fed back to the user. Therefore, it is possible to prevent a biosignal from being measured wrongly in a state where the user's body is touching the electrodes incorrectly, thereby significantly improving reliability of biosignal measurement and medical diagnosis.

In addition, according to the present invention, which parts of the user's body were in contact with the electrodes formed in the biosignal measurement apparatus, and the biosignal is corrected based on the identified parts. This makes more accurate biosignal measurement possible.

### [Description of Drawings]

FIG. 1 is a diagram for schematically explaining the principle behind how an electric current flows through the human body;
FIG. 2 illustrates an embodiment of a biosignal measurement apparatus according to the present invention;
FIG. 3 illustrates a modified embodiment of the biosignal measurement apparatus of FIG. 2;
FIG. 4 illustrates waveform changes in an electrocardiogram (ECG) signal according to a degree to which the human body touches a plurality of electrodes formed in the biosignal measurement apparatus; and
FIG. 5 illustrates example biosignal information displayed on a display module of the biosignal measurement apparatus according to the present invention.

### [Mode for Invention]

Hereinafter, exemplary embodiments of the present invention will be described in detail with reference to the accompanying drawings to enable those skilled in the art to easily implement the invention.

For a clear understanding of the present invention, a detailed description of components and features irrelevant to the present invention will be omitted. Like reference numerals refer to like components throughout the specification. In addition, the shape and size of each component illustrated in the drawings are merely intended for ease of description, but the present invention is not necessarily limited to the shape and size. That is, specific shapes, structures and characteristics described herein may be implemented as modified from one embodiment to another without departing from the spirit and scope of the invention. Furthermore, it shall be understood that the locations or arrangements of individual components within each embodiment may also be modified without departing from the spirit and scope of the invention. Therefore, the following detailed description is not to be taken in a limiting sense, and the scope of the invention is to be taken as encompassing the scope of the appended claims and all equivalents thereof.

The human body has a certain degree of conductivity despite its high resistance. Thus, the human body can be considered as a conducting wire. The conductivity of the human body enables the human body to function as a medium such as an electric wire, and an electric current flowing through the human body can be used to transmit or receive information without power consumption.

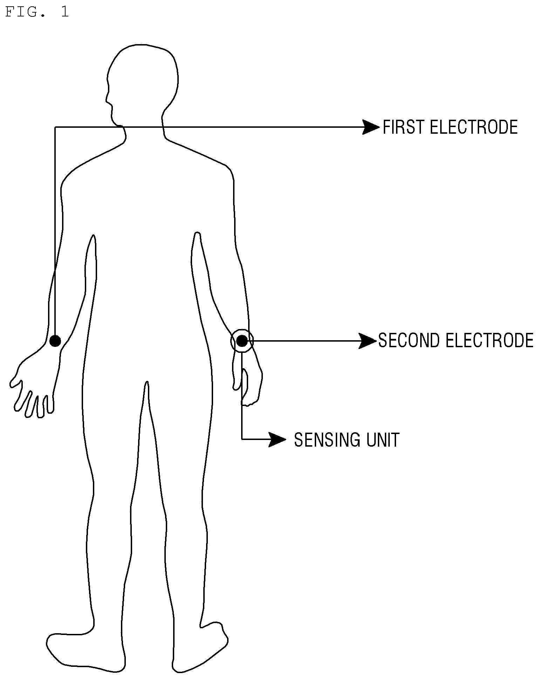

That is, when two electrodes having different electric potentials come into contact with the human body, a circuit is connected between the two electrodes by the human body, and an electric current flows in the circuit. For example, referring to FIG. 1, when an electrode (a first electrode) having a relatively high electric potential contacts one hand of the human body and when another electrode (a second electrode) having a relatively low electric potential contacts the other hand of the human body, a circuit may be connected through the human body, and an electric current may flow in the circuit. Here, an electrical signal (e.g., a voltage or an electric current) generated by the connection of the circuit may be sensed by a sensing unit connected to the electrodes. Thus, it is a feature of the present invention to utilize a plurality of electrodes formed in a biosignal measurement apparatus as touch sensors by using the flow of an electric current between the electrodes caused by contact with the human body.

The present invention utilizes a plurality of electrodes formed in a biosignal measurement apparatus as touch sensors based on the following basic principles.
(1) An electric current flows between two electrodes when one of the electrodes has a higher or lower electric potential than the other one.
(2) An open circuit is formed between the two electrodes when only one of the two electrodes contacts the human body or when none of the two electrodes contacts the human body. Therefore, no electric current flows between the two electrodes.
(3) When both of the two electrodes contact the human body, the human body serves as a conducting wire. Therefore, an electric current flows from an electrode having a relatively high electric potential to an electrode having a relatively low electric potential. Here, an electrical signal (e.g., a voltage or an electric current) generated by the human body may be sensed by a sensing unit connected to the electrodes. The sensing unit may be configured to sense an electrical signal generated by the human body by converting the electrical signal (the voltage or the electric current) as it is or by converting the electrical signal according to a range based on a set threshold.
(4) An electrical signal generated by the contact of the electrodes with the human body varies according to the degree of contact between the electrodes and the human body. When the human body touches the electrodes hard, a high electrical signal is generated. When the human body touches the electrodes lightly, a low electrical signal is generated. Therefore, the magnitude of an electrical signal (a voltage or an electric current) measured by the sensing unit indicates the degree to which the human body touches the electrodes.
(5) If one of the two electrodes is always in contact with the human body, whether an electric current flows through the human body is determined based on whether the human body has touched the other electrode. Therefore, a touch sensor function may be performed based on whether the human body has touched an electrode which is always not in contact with the human body.

Technical features of the present invention will hereinafter be described in greater detail using exemplary embodiments of a biosignal measurement apparatus according to the present invention.

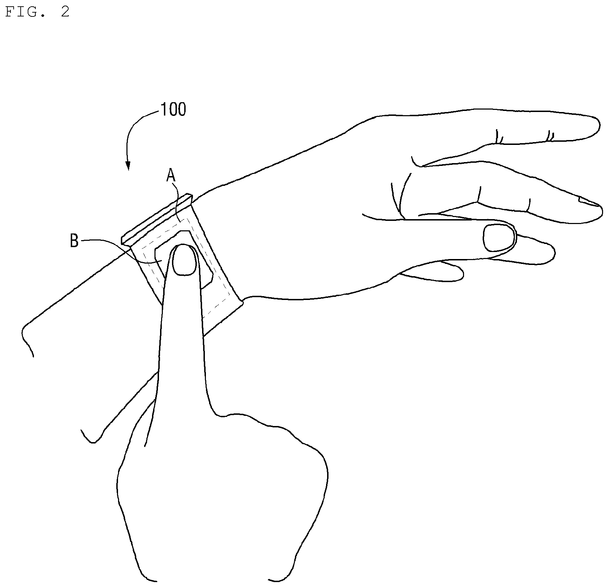

FIG. 2 illustrates an embodiment (a wristwatch apparatus) of a biosignal measurement apparatus according to the present invention. Referring to FIG. 2, the biosignal measurement apparatus (the wristwatch apparatus) according to the embodiment of the present invention may be configured similarly to the above-described conventional biosignal measurement apparatus. Specifically, the biosignal measurement apparatus 100 according to the embodiment of the present invention is formed in the shape of a wristwatch so that it can be worn around a user's wrist. The biosignal measurement apparatus 100 includes a first electrode A for biosignal measurement on the back (an inner surface that contacts the wrist wearing the biosignal measurement apparatus 100) thereof and a second electrode B for biosignal measurement on the front (an outer surface that does not contact the wrist wearing the biosignal measurement apparatus 100) thereof. The first electrode A and the second electrode B included in the biosignal measurement apparatus 100 form an electrocardiogram (ECG) sensor module for measuring the user's ECG signal. For example, in a state where the first electrode A is in contact with the user's wrist wearing the biosignal measurement apparatus 100, if the user touches the second electrode B with another part (such as a finger) of his or her body, the user's ECG signal can be measured by the first electrode A and the second electrode B.

According to an embodiment of the present invention, the second electrode B can also be formed on a display screen of a display module formed on the front of the biosignal measurement apparatus. In this case, in a state where the first electrode A is in contact with the user's wrist, if the user touches the display screen of the display module having the second electrode B with another part (such as a finger) of his or her body, the user's ECG signal can be measured by the first electrode A and the second electrode B. Here, the display screen of the display module may be configured as a touchscreen.

In addition, the biosignal measurement apparatus 100 according to the present invention may include at least one photoplethysmography (PPG) sensor module (photo sensor module) for measuring PPG and/or saturation of peripheral oxygen (SpO₂). As described above, PPG and SpO₂ can be measured by irradiating light generated by a light emitting unit of the PPG sensor module (the photo sensor module) to the user's fingertip or toe tip and observing a change in the amount of light transmitted or reflected by the human body and then received by a light receiving unit. The PPG sensor module can be installed at any location. However, the PPG sensor module may preferably be formed at a location where the electrodes of the ECG sensor module are formed. If the PPG sensor module is formed at the location where the electrodes of the ECG sensor module are formed, PPG and SpO₂ signals as well as the ECG signal can be measured.

The PPG sensor module for measuring PPG and/or SpO₂ may include a light emitting unit (not illustrated) and a light receiving unit (not illustrated). The light emitting unit includes a red light-emitting diode which generates red light having a wavelength of approximately 660 nm and an infrared light-emitting diode which generates infrared light having a wavelength of approximately 940 nm. The light receiving unit includes a photo diode and/or a photo transistor. For example, the biosignal measurement apparatus 100 according to the embodiment of the present invention may be configured to include a PPG sensor module, which includes a light emitting unit formed as an infrared light-emitting diode and a light receiving unit formed as a photo diode, at a location where the second electrode B is formed.

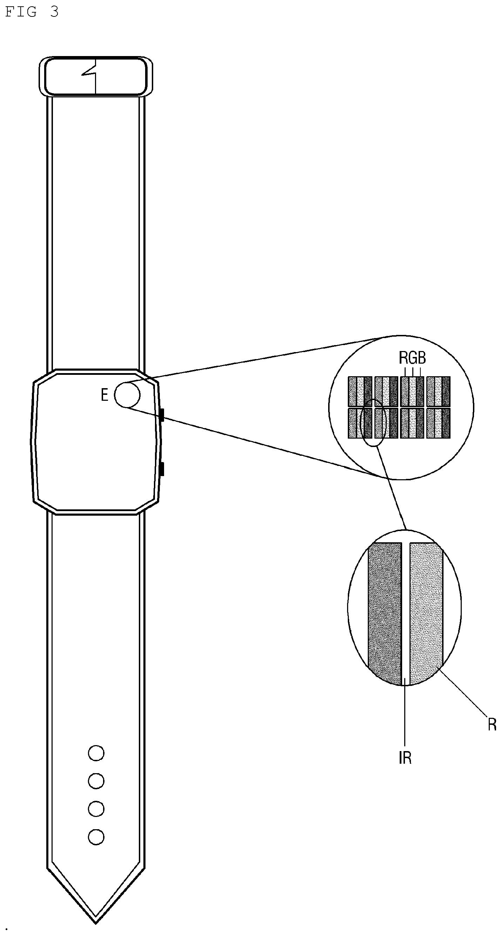

The PPG sensor module for measuring PPG and/or SpO₂ can also be implemented using the display module formed on the front of the apparatus. FIG. 3 illustrates an exemplary embodiment in which the PPG sensor module is configured using the display module of the biosignal measurement apparatus 100.

For example, referring to FIG. 3, the biosignal measurement apparatus 100 includes a measurement area E for measuring the user's biosignals (PPG and/or SpO₂) in a part of the display module. As described above, red light should be irradiated to the human body in order to measure PPG, and red light and infrared light should be irradiated to the human body in order to measure SpO₂. To this end, the biosignal measurement apparatus 100 may include, as illustrated in FIG. 3, an infrared subpixel IR for forming infrared light in addition to RGB subpixels (a red subpixel R for forming red light, a green subpixel G for forming green light, and a blue subpixel B for forming blue light) typically used in a pixel structure of the measurement area E of the display module. In this configuration, red light and infrared light can be irradiated to an area E by the red subpixel R and the infrared subpixel IR included in the pixel structure of the measurement area E of the display module. The red light and the infrared light irradiated to the area E may function as the light emitting unit of the PPG sensor module (the photo sensor module) for measuring PPG and/or SpO₂. In addition, the light receiving unit may further be provided in the measurement area E of the display module to receive light irradiated by the red subpixel R and the infrared subpixel IR and then reflected by the human body. If the biosignal measurement apparatus 100 configured as described above is used, biosignals such as PPG and/or SpO₂ can be measured using the display module of the biosignal measurement apparatus 100 without the need to form additional photo sensors in the biosignal measurement apparatus 100.

The biosignal measurement apparatus 100 configured as described above can be used to measure various biosignals such as the user's ECG, PPG and SpO₂ using the sensor modules (the ECG sensor module, the PPG sensor module, etc.) included therein. In addition, biosignal information thus measured can be stored in a storage device (not illustrated) included in the biosignal measurement apparatus 100 or analyzed and processed by a control unit (not illustrated). For example, ECG, PPG and SpO₂ measured by the ECG sensor module and the PPG sensor module can be used to estimate the user's blood pressure in real time. A method of measuring and analyzing a biosignal and estimating blood pressure based on the measured biosignal is disclosed in Korean Patent Application Nos. 2013-116158 and 2012-54770 filed by the present inventor. It should be understood that the above disclosures are incorporated herein by reference in their entirety.

The biosignal measurement apparatus 100 according to the present invention is configured to utilize a plurality of electrodes (the first electrode A and the second electrode B) formed therein for biosignal measurement as described above as touch sensors for executing other functions.

First, the electrodes (the first electrode A and the second electrode B) formed in the biosignal measurement apparatus 100 according to the present invention can be used to measure a biosignal (e.g., the ECG signal), which is their primary function. For example, in a state where a biosignal measurement program included in the biosignal measurement apparatus 100 has been made to run by the user, if the user's body touches the electrodes formed in the biosignal measurement apparatus 100, the user's ECG signal can be measured based on signals from both electrodes.

The electrodes (the first electrode A and the second electrode B) formed in the biosignal measurement apparatus 100 according to the present invention can be used as touch sensors for executing functions other than the above biosignal measurement function. For example, in a state where the biosignal measurement program of the biosignal measurement apparatus 100 is not running, if the user's body touches the electrodes (the first electrode A and the second electrode B), the biosignal measurement apparatus 100 may execute various functions by sensing the touch of the user's body with the electrodes.

For example, if the user's body touches the electrodes (the first electrode A and the second electrode B) in a state where there is an incoming call to the biosignal measurement apparatus 100, the biosignal measurement apparatus 100 may automatically reject the incoming call. The biosignal measurement apparatus 100 according to the present invention may also be configured to execute a different function according to a period of time during which the user's body touches the electrodes (the first electrode A and the second electrode B). For example, in a state where the biosignal measurement program is not running, if the user's body touches the electrodes (the first electrode A and the second electrode B) for less than a first reference time (e.g., 3 seconds), an alarm (a first function) may be set after a certain time. If the user's body touches the electrodes (the first electrode A and the second electrode B) for the first reference time or longer and less than a second reference time (e.g., 5 seconds), a recoding function (a second function) may be executed. If the user's body touches the electrodes (the first electrode A and the second electrode B) for the second reference time or longer, the biosignal measurement function may be executed. In addition to the above examples, functions executed by the contact of the user' body with the electrodes or reference times for distinguishing the functions can be variously and arbitrarily set/modified as the user desires.

For more accurate body signal measurement, the biosignal measurement apparatus 100 according to the present invention may also be configured to determine whether the user's body has properly touched the electrodes to a degree appropriate for biosignal measurement and feed the determination result back to the user.

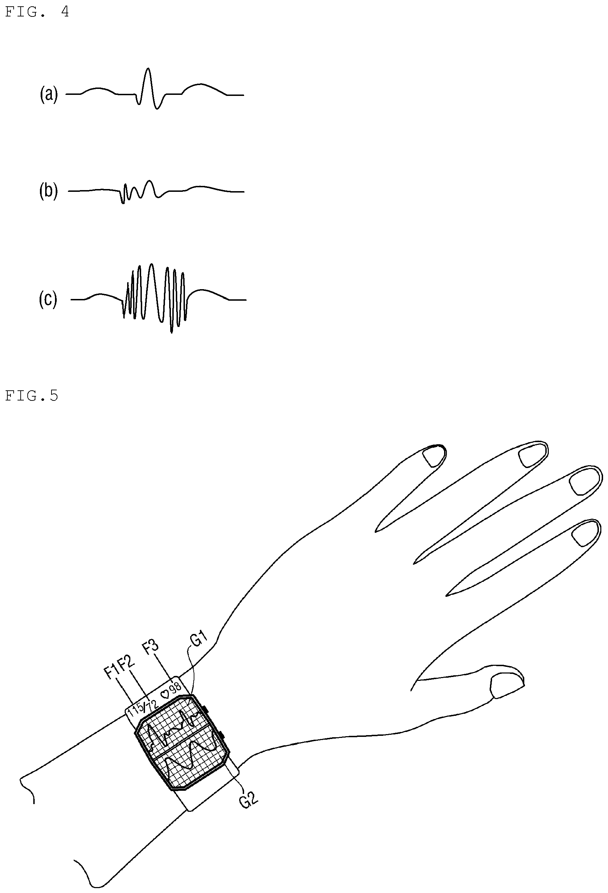

Specifically, the user's biosignal (ECG signal) has a different waveform according to the degree to which the user's body touches the electrodes. FIG. 4 illustrates a waveform of a biosignal according to the degree to which the user's body touches the electrodes. For example, when the user's body touches the electrodes to an appropriate degree for biosignal measurement, the biosignal has a normal waveform as illustrated in (a) of FIG. 4. However, when the user's body does not touch the electrodes to the appropriate degree for biosignal measurement, the biosignal has a different waveform from the normal waveform. For example, when the user's body touches the electrodes too lightly, the biosignal has a waveform illustrated in (b) of FIG. 4. When the user's body touches the electrodes too hard, an electromyogram (EMG) signal may be introduced. As a result, the biosignal has a waveform illustrated in (c) of FIG. 4. The biosignal measured when the user's body is touching the electrodes to an inappropriate degree may lead to misdiagnosis of the user's health condition.

To prevent this problem, the present invention determines whether the degree to which the user's body touches the electrodes is appropriate. When determining that the user's body has not touched the electrodes to an appropriate degree for biosignal measurement, the present invention informs the user of the determination result. This ensures biosignal measurement according to a normal protocol, thereby improving the reliability of biosignal measurement.

For example, the biosignal measurement apparatus 100 according to the present invention may be configured to determine whether the user's body has touched the electrodes appropriately based on the magnitude of an electrical signal (e.g., a voltage or an electric current) measured by the sensing unit connected to the electrodes. Specifically, if the electrical signal measured by the sensing unit is within a preset range (a range appropriate for biosignal measurement), the biosignal measurement apparatus 100 may determine that the user's body has touched the electrodes appropriately and thus measure the user's biosignal. On the other hand, if the electrical signal measured by the sensing unit is outside the preset range (the range appropriate for biosignal measurement), the biosignal measurement apparatus 100 may inform the user that the user's body has touched the electrodes inappropriately through an image display alarm of the display module or a sound alarm, thereby inducing the user to correctly touch the electrodes again. Therefore, biosignal measurement can be performed in the state of correct touch. The above configuration can prevent a biosignal from being measured wrongly in a state where the user's body is touching the electrodes incorrectly, thereby preventing the misdiagnosis of the user's health condition and significantly improving the reliability of biosignal measurement and medical diagnosis.

In addition, the biosignal measurement apparatus 100 according to the present invention may be configured to further improve the accuracy of biosignal measurement by appropriately correcting a measured biosignal. Generally, a biosignal has a different value according to parts of the human body which touch sensors. Therefore, to obtain more accurate biosignal information, which parts of the human body have generated a measured biosignal should be identified, and the measured biosignal should be corrected based on the identified parts of the human body. To this end, the biosignal measurement apparatus 100 according to the present invention may be configured to correct a measured biosignal by identifying parts of the human body which touched the sensors (the electrodes). For example, the biosignal measurement apparatus 100 formed in the shape of the wristwatch apparatus as illustrated in FIG. 2 typically measures a biosignal (ECG signal) in a state where the first electrode A is in contact with the user's wrist and where the second electrode B is in contact with the user's finger. Therefore, the biosignal measurement apparatus 100 (the wristwatch apparatus) according to the present invention may be configured to correct the biosignal based on the assumption that the biosignal was measured in a state where the user's wrist was touching the first electrode A and where the user's finger was touching the second electrode B. In this configuration, since parts of the user's body which generated the biosignal are identified more accurately, a more accurate biosignal can be obtained.

Biosignal information measured and/or estimated as described above may be provided to the user in real time through the display screen of the display module formed on the front of the biosignal measurement apparatus 100. For example, referring to FIG. 5, various numerical information such as systolic blood pressure F1, diastolic blood pressure F2 and pulse F3 or a graph showing changes in an ECG signal G1, a PPG signal G2, etc. may be displayed on the display screen of the display module. In addition, the display screen of the display module may display a graph showing the user's real-time blood pressure information estimated based on the above biosignals. The content or style of information displayed on the display module may be selected or modified by the user.

While the present invention has been described above using particular examples, including specific components, by way of limited embodiments and drawings, it is to be appreciated that these are provided merely to aid the overall understanding of the present invention, the present invention is not to be limited to the embodiments above, and various modifications and alterations can be made from the disclosures above by a person having ordinary skill in the technical field to which the present invention pertains.

Therefore, the spirit of the present invention must not be limited to the embodiments described herein, and the scope of the present invention must be regarded as encompassing not only the claims set forth below, but also their equivalents and variations.

## Claims

1. A biosignal measurement apparatus capable of measuring a user's biosignal in real time, the biosignal measurement apparatus comprising:
a plurality of electrodes which are separated from each other and configured to measure a biosignal through contact with a user's body; and
a sensing unit which is electrically connected to the electrodes and measures an electrical signal formed in a circuit connected to the electrodes,
wherein the electrodes are also configured to be utilized as touch sensors for executing functions other than a biosignal measurement function of the biosignal measurement apparatus, and the biosignal measurement apparatus is configured to execute a different function according to whether the user's body has touched the electrodes and a period of time during which the user's body touches the electrodes.

2. The biosignal measurement apparatus of claim 1, being configured to utilize the electrodes as touch sensors by determining whether the user's body has touched the electrodes, based on whether the electrical signal has been sensed by the sensing unit.

3. The biosignal measurement apparatus of claim 1, wherein the electrodes of the biosignal measurement apparatus are configured to function as electrodes for biosignal measurement in a state where a biosignal measurement program is running in the biosignal measurement apparatus and to function as touch sensors for executing functions other than biosignal measurement in a state where the biosignal measurement program is not running.

4. The biosignal measurement apparatus of claim 1, being configured to execute a first function other than the biosignal measurement program when the user's body touches the electrodes for less than a first reference time in the state where the biosignal measurement program is not running, a second function other than the biosignal measurement program when the user's body touches the electrodes for the first reference time or longer and less than a second reference time in the state where the biosignal measurement program is not running, and the biosignal measurement program when the user's body touches the electrodes for the second reference time or longer in the state where the biosignal measurement program is not running.

5. The biosignal measurement apparatus of claim 4, wherein the first reference time, the second reference time, the first function, and the second function are configured to be set and modified by the user.

6. The biosignal measurement apparatus of claim 1, being configured to determine a degree to which the user's body touches the electrodes and, when determining that the degree to which the user's body touches the electrodes is outside a range for biosignal measurement, inform the user of the determination result.

7. The biosignal measurement apparatus of claim 1, being a wristwatch apparatus which can be worn around the user's wrist, wherein the electrodes comprise a first electrode formed on a surface which contacts the user's wrist wearing the biosignal measurement apparatus and a second electrode disposed on a different surface from the first electrode.

8. The biosignal measurement apparatus of claim 7, wherein the second electrode is disposed on a display screen of a display module formed on a front of the biosignal measurement apparatus.

9. The biosignal measurement apparatus of claim 7, being configured to correct a biosignal based on the assumption that the biosignal was measured in a state where the first electrode A was in contact with the user's wrist and where the second electrode B was in contact with the user's finger.

10. The biosignal measurement apparatus of claim 1, wherein the electrodes form an electrocardiogram (ECG) sensor module for measuring the user's ECG signal.

11. The biosignal measurement apparatus of claim 1, wherein the sensing unit is configured to sense the electrical signal by converting a value of a voltage or an electric current generated by the human body as it is or by converting the voltage or the electric current generated by the human body according to a range based on a set threshold.
